# EUROPEAN PATENT APPLICATION

(11) **EP 4 140 470 A2**
(43) Date of publication of application: **01.03.2023**
(21) Application number: 22188658.3
(22) Date of filing: 04.08.2022
(51) Int. Cl.: A61K 8/34, A61K 8/66, A61Q 19/00, B65D 81/32

(54) **COSMETIC COMPOSITION FOR PREVENTING ENZYME DAMAGE AND COSMETIC CONTAINER STORING THE SAME**

(30) Priority: 24.08.2021 KR 20210111545
(71) Applicant: Amorepacific Corporation, Seoul 04386 (KR)
(72) Inventor: SEUNGHA, Yang, 17074 Gyeonggi-do (KR); KYUNGSUP, Han, 17074 Gyeonggi-do (KR); EUNMI, Kim, 17074 Gyeonggi-do (KR); BYUNGFHY, Suh, 17074 Gyeonggi-do (KR); SOONAE, An, 17074 Gyeonggi-do (KR); SOWOONG, Choi, 17074 Gyeonggi-do (KR); JOONYOUNG, Hwang, 17074 Gyeonggi-do (KR)
(74) Representative: Germain Maureau

(57) **Abstract**

This disclosure relates to a cosmetic composition capable of preventing enzyme damage, and a cosmetic container for storing the same. One aspect of this disclosure provides a cosmetic composition capable of preventing enzyme damage, the composition including a first content disposed in a first space; a second content which is disposed in a second space separated from the first space, and which includes an enzyme and a polyol, wherein a cosmetic material is produced by mixing the first content and the second content.

## Description

### TECHNICAL FIELD

This disclosure relates to a cosmetic composition for preventing enzyme damage, and a cosmetic container for storing the same.

### BACKGROUND

Enzymes act as biocatalysts that enable rapid and accurate biochemical reactions which occur inside living organisms. Recently, studies on cosmetics for whitening and wrinkle improvement using these enzymes are being actively conducted.

However, since enzymes are activated only under a specific condition such as temperature, pH or the like, there is a problem in that it is difficult to maintain the activity in the cosmetic formation.

Since it is difficult to maintain the enzyme in an activity, some methods have been conventionally proposed in which enzymes are stored in a dry state such as in powder form, and then moisture is added to them when being used.

However, using enzymes in a dry state is disadvantageous in that it is cumbersome to add water to the enzymes to reactivate them, and in that a separate device for homogeneous mixing is required. Additionally, there was a problem that if a separate device for mixing is not optimized, the enzyme could be damaged during the mixing process.

Meanwhile, a method of adding enzymes to a general cosmetic formulation containing moisture has been tried, but there has been a problem that the enzyme is destroyed by the surfactant and other effect materials included in the cosmetic formulation.

### SUMMARY

The embodiments of the disclosure, which have been conceived to address above-described problems, are to provide a cosmetic composition in which enzyme activity can be maintained even when stored for a long period of time, and a cosmetic container for storing the same.

This disclosure is to provide a cosmetic composition capable of avoiding enzyme damage during a mixing process of a first content and a second, and of being discharged in a state in which the enzyme activity is maintained even in an emulsion or solubilization cosmetic material after they have been mixed, and to provide a cosmetic container for the same.

According to an aspect of the present invention, there is provided a cosmetic composition capable of preventing enzyme damage, the composition comprising: a first content disposed in a first space; a second content which is disposed in a second space separated from the first space, and which includes an enzyme and a polyol, wherein a cosmetic material is produced by mixing the first content and the second content.

Further, there is provided a cosmetic composition, wherein the polyol includes at least one selected from a group consisting of ethylene glycol, propylene glycol, dipropylene glycol, 1,3-butylene glycol, diglycerin, 1,3-propanediol, glycerin, methylpropanediol, ethylhexanediol, 1,2-hexanediol, isoprene glycol, ethylalcohol, pentylene glycol, and isopentyldiol.

Further, there is provided a cosmetic composition, wherein the polyol is provided in an amount of 40% or more based on the total weight of the second content.

Further, there is provided a cosmetic composition, wherein the enzyme is provided as a protease.

Further, there is provided a cosmetic composition, wherein the protease includes at least one selected from a group consisting of cysteine protease, serine protease, threonine protease, glutamate protease, aspartic acid protease, metalloprotease, asparagine protease, keratinase, papain, and lipase.

Further, there is provided a cosmetic composition, wherein the second content further includes a thickener having a viscosity of 600 cps to 8,000 cps.

Further, there is provided a cosmetic composition, wherein the thickener includes at least one selected from a group consisting of gelatin, gellan gum, guar gum, xanthan gum, locust bean gum, alginic acid, arabian gum, carrageenan, pectin, cellulose, mannan, carbomer, sodium polyacrylate, polyacrylic acid, polyacrylate crosspolymer, hydroxyethyl acrylate/sodium acryloyldimethyl taurate copolymer, ammonium acryloyldimethyltaurate/vp copolymer, sodium polyacryloyldimethyl taurate, acrylate/C10-30 alkyl acrylate crosspolymer, ammonium acryloyldimethyltaurate/beheneth-25 methacrylate crosspolymer, poly vinyl alcohol, and glyceryl acrylate/acrylic acid copolymer.

Further, there is provided a cosmetic composition, wherein the first content includes at least one of a moisturizing agent, a surfactant, and a thickener.

Further, there is provided a cosmetic composition, wherein the first content includes a surfactant, and the second content including the enzyme does not include a surfactant.

Further, there is provided a cosmetic composition, wherein the cosmetic material produced by mixing the first content and the second content includes emulsion or solubilization cosmetic material.

According to an aspect of the present invention, there is provided a cosmetic container for storing a cosmetic composition, the container comprising: a first container providing a first space in which a first content can be stored; a second container providing a second space in which a second content can be stored; and a channel part provided with a microfluidic channel capable of forming a cosmetic material by mixing the first content and the second content, wherein the Reynolds numbers of the first content, the second content, and the cosmetic material which pass through the microfluidic channel of the channel part are provided to be 1 or less, the cosmetic material being a mixture of the first and second contents.

Further, there is provided a cosmetic container, wherein the microfluidic channel has a height of 1 mm or less.

Further, there is provided a cosmetic container, wherein the fluid entry velocities of the first content, the second content, and the cosmetic material which pass through the microfluidic channel of the channel part are provided to be 0.9 m/s or less.

Further, there is provided a cosmetic container, wherein the channel part includes: a first injection hole for receiving the first content from the first container; a second injection hole for receiving the second content from the second container; a confluence part where the first content and the second content are joined to produce the cosmetic material; and a plurality of mixing parts extending from the confluence part so as to change the progress directions of the first content, the second content, and the cosmetic material.

Further, there is provided a cosmetic container, wherein a number of the mixing parts is three.

Further, there is provided a cosmetic container, wherein the mixing of the first content and the second content in the microfluidic channel of the channel part occurs while the interfaces of the first content and the second content overlap each other.

Further, there is provided a cosmetic container, further comprising another first container provided with another first space in which a first content can be stored, wherein the channel part includes: a first injection hole for receiving the first content from the first container; an additional injection hole for receiving the first content from said another first container; a second injection hole for receiving the second content from the second container; a confluence part in which the first and second contents are joined to form the cosmetic material; and a plurality of mixing parts extending from the confluence part so as to change the progress directions of the first content, the second content, and the cosmetic material.

The cosmetic composition for preventing enzyme damage and the cosmetic container storing the same according to an embodiment of this disclosure are advantageously capable of maintaining the activity of the enzyme even during long-term storage.

Additionally, there is an advantage of being capable of avoiding enzyme damage during the mixing process of the first content and the second content, and of being discharged in a state in which the enzyme activity is maintained even in an emulsion or solubilization cosmetic material after they have been mixed.

### BRIEF DESCRIPTION OF DRAWINGS

The above and other objects, features and advantages of this disclosure will become more apparent to those skilled in the art by describing exemplary embodiments thereof in detail with reference to the accompanying drawings, in which:
FIG. 1 is a photograph showing the degree of enzyme activation when the first content and the second content of Experimental Example 1 are mixed and discharged by means of a cosmetic container according to an embodiment of this disclosure;
FIG. 2 is a photograph of Comparative Example 1 showing the degree of enzyme activation when the first and second contents are mixed by means of a general mixer;
FIG. 3 is a perspective view schematically showing a cosmetic container 10 according to another embodiment of this disclosure;
FIG. 4 shows an exploded perspective view of the cosmetic container 10 of FIG. 3;
FIG. 5 is a cross-sectional view showing the microfluidic channel 300 formed in the channel part 400 of FIG. 4;
FIG. 6 is a perspective view showing a state in which an inner cap 800 and an outer cap 900 are separated from the cosmetic container 10 of FIG. 3;
FIG. 7 is an exploded perspective view showing the more detailed configuration of an operation unit 500 in the cosmetic container 10 of FIG. 3;
FIG. 8 is a perspective view showing a button lever 530 of the cosmetic container 10 of FIG. 7;
FIG. 9 is a side cross-sectional view of the button lever 530 of FIG. 8;
FIG. 10 is a side view showing a pressurization panel of the operation unit 500 of FIG. 4;
FIG. 11 represents an exploded perspective view of a cosmetic container according to another embodiment of this disclosure; and
FIG. 12 is a diagram representing a cross-section of a channel part 400' provided in the cosmetic container of FIG. 11.

### DETAILED DESCRIPTION OF EXEMPLARY EMBODIMENTS

Hereinafter, specific exemplary embodiments of this disclosure will be described in detail with reference to the drawings. Additionally, it is noted that in the description of the disclosure, the detailed description for known related configurations or functions may be omitted when it is deemed that such description may obscure essential points of the disclosure.

The cosmetic composition for preventing enzyme damage according to an embodiment of this disclosure may include a first content disposed in a first space; and a second content which is disposed in a second space separated from the first space, and which includes an enzyme and a polyol.

Additionally, an emulsion may be produced by mixing the first and second contents, and the mixing of the first and second contents may occur in a channel part 400 of a cosmetic container 10 to be described later.

In this embodiment, the first content including a component (e.g., surfactant) capable of damaging an enzyme is disposed in the first space, and the second content including the enzyme is disposed in a second space separated from the first space. As such, by physically separating the enzyme and the component (e.g., surfactant) that can damage the enzyme, damage to the enzyme can be prevented or minimized.

The first content may include components for producing cosmetic material (e.g., emulsion cosmetic material such as O/W emulsion and W/O emulsion, solubilization cosmetic material which is a formulation with little oil), and the second content may include an enzyme and a component for preventing damage to the enzyme. Additionally, the second content does not include large amounts of surfactants, ions, salts, or the like that can damage the enzyme.

Additionally, the emulsion (or emulsion cosmetic material) produced by mixing the first content and the second content may include an O/W emulsion and a W/O emulsion. Whether the O/W emulsion or the W/O emulsion is produced may vary depending on the ratio of the oil-based fluid and the water-based fluid included in the first and second contents. For example, when mixing of the first content and the second content occurs in the channel part 400 to be described later, in a case where the water-based fluid is mixed in a greater proportion than the oil-based fluid, an O/W emulsion may be produced, while in the opposite case, a W/O emulsion may be produced.

Here, the O/W emulsion may be understood as including emulsified particles in which a hydrophobic fluid such as oil is uniformly dispersed in a particle state in a hydrophilic fluid such as water, and the W/O emulsion may be understood as including emulsified particles in which the hydrophilic fluid is uniformly dispersed in a particle state in a hydrophobic fluid.

Additionally, a solubilization cosmetic material may be understood as a cosmetic material in which a small amount of oil that does not dissolve in water is dissolved in a transparent state using the micelle-forming action of a solubilizing agent (e.g., surfactant).

Although it is described as an example in this embodiment that an emulsion is produced by mixing the first content and the second content, the spirit of this disclosure is not limited thereto, and may include producing a solubilization cosmetic material by mixing the first and second contents.

Additionally, examples of the solubilization cosmetic material produced by mixing the first content and the second content may include skin lotion, essence, perfume, and the like.

The second content may include an enzyme, a polyol, and a thickener.

The enzyme may include a protease. For example, the protease may include at least one of cysteine protease, serine protease, threonine protease, glutamate protease, aspartic acid protease, metalloprotease, asparagine protease, keratinase, papain, and lipase.

Additionally, the second content may include a polyol.

The polyol may be understood as a multifunctional alcohol having two or more hydroxyl groups (-OH) in a molecule. The polyol may include glycol having two hydroxyl groups, glycerol having three hydroxyl groups, and pentaerythritol having four hydroxyl groups.

For example, the polyol may include at least one selected from a group consisting of ethylene glycol, propylene glycol, dipropylene glycol, 1,3-butylene glycol, diglycerin, 1,3-propanediol, glycerin, methylpropanediol, ethylhexanediol, 1,2-hexanediol, isoprene glycol, ethylalcohol, pentylene glycol, and isopentyldiol.

Additionally, the polyol may be provided in an amount of 40% or more based on the total weight of the second content. For example, the polyol may be provided in a proportion of 40% to 80% based on the total weight of the second content.

As such, by including the polyol in a specific ratio or more together with the enzyme, water activity can be reduced. Here, water activity is a factor affecting enzyme activity, and as the water activity decreases, the enzyme can be stored stably.

Additionally, the second content may further include a thickener together with the above-described enzyme and polyol.

The thickener may exhibit a viscosity of 600 cps to 8,000 cps. Preferably, the viscosity of the thickener may exhibit 660 cps to 8,000 cps, and more preferably, the viscosity of the thickener may exhibit 1000 cps to 8,000 cps. For example, the viscosity of the thickener included in the second content may exhibit 3000 cps to 4,000 cps.

In the channel part 400 of the cosmetic container to be described later, the first content and the second content are mixed to produce an emulsion. At this time, the flow of the first content and the second content in the channel part 400 should not affect the activity state of the enzyme.

In this embodiment, by adjusting the viscosity range of the thickener included in the second content to the above-described range, the first content and the second content can be mixed to form an emulsion without affecting the activity state of the enzyme. Amore detailed description of the mixing of the first content and the second content occurring in the channel part 400 will be described later.

Additionally, the thickener included in the second content may include at least one selected from a group consisting of Gelatin, Gellan Gum, Guar Gum, Xanthan Gum, Locust Bean Gum, Alginic acid, Arabian Arabic Gum, Carrageenan, Pectin, Cellulose, Mannan, Carbomer, Sodium Polyacrylate, Polyacrylic Acid, Polyacrylate crosspolymer, Hydroxyethyl acrylate/Sodium acryloyldimethyl taurate Copolymer, Ammonium acryloyldimethyltaurate/vp copolymer, Sodium polyacryloyldimethyl taurate, Acrylate/C10-30 Alkyl acrylate Crosspolymer, Ammonium acryloyldimethyltaurate/beheneth-25 methacrylate crosspolymer, Poly vinyl alcohol, and glyceryl acrylate/acrylic acid copolymer.

The first content may include a component for being mixed with the second content to produce an emulsion or solubilization cosmetic material. For example, the first content may include a moisturizing agent, a surfactant, a thickener, and an adjuvant emulsifier.

These components included in the first content are exemplary, and the first content may include every known component for producing an emulsion or solubilization cosmetic material.

The moisturizing agent may include at least one selected from a group consisting of Trehalose, Fructose, Sucrose, Sorbitol, Maltitol, Panthenol, Raffinose, Urea, Betaine, Glycereth-26, Methyl Gluceth-20, PEG-8, PEG-32, PEG-75, PEG/PPG/POLYBUTYLENE GLYCOL-8/5/3 GLYCERIN, ethylene glycol, propylene glycol, dipropylene glycol, 1,3-butylene glycol, diglycerin, 1,3-propanediol, glycerin, methylpropanediol, ethylhexanediol, 1,2-hexanediol, isoprene glycol, ethylalcohol, pentylene glycol, and isopentyldiol.

The surfactant included in the first content may include at least one selected from a group consisting of Sorbitan Monostearate, Sorbitan Sesquioleate, Polysorbate 20, Polysorbate 60, Polysorbate 80, PEG-40 Stearate, PEG-100 Stearate, PEG-60 Glyceryl Isostearate, PEG 75- Stearate, Ceteth-20, Steareth-20, Cetearyl Glucoside, Arachidyl Glucoside, Lauryl Glucoside, Sucrose Polystearate, Sucrose Laurate, Polyglyceryl-3 Methylglucose Distearate, Polyglyceryl-10 Stearate, Lecithin, Sodium Stearoyl Glutamate, Glyceryl Stearate Sheet Glyceryl Stearate Citrate, Potassium Cetyl Phosphate, Sodium Methyl Stearoyl Taurate, Inulin Lauryl Carbamate, Ceteareth-12, PEG-5 Rapeseed Sterol, Methoxy PEG-114/Polyepsion Caprolactone, Cetyl PEG/PPG 10/1 Dimethicone Triglyceride, PEG-10 Dimethicone, Lauryl PEG-9 Polydimethylsiloxyethyl Dimethicone, Polyglyceryl-2 Dipolyhydroxystearate, PEG-60 Hydrogenated Castor Oil, PEG-30 Hydrogenated Castor Oil, Octyldodeceth-16, Octyldodeceth-25, Bis-PEG-18 Methyl Ether Dimethyl Silane, Polyglyceryl-6 Caprylate, and Polyglyceryl-4 Caprate.

Additionally, the adjuvant emulsifier included in the first content may include at least one selected from a group consisting of Cetearyl Alcohol, Cetyl Alcohol, Stearyl Alcohol, Behenyl Alcohol, Glyceryl Stearate, and Stearic Acid.

Additionally, the thickener included in the first content may include the same components as the thickener included in the above-described second content.

Specifically, the thickener included in the first content may exhibit a viscosity of 600 cps to 8,000 cps. Preferably, the viscosity of the thickener may exhibit 660 cps to 8,000 cps, and more preferably, the viscosity of the thickener may exhibit 1000 cps to 8,000 cps, and further more preferably, the viscosity of the thickener may exhibit 3000 cps to 4,000 cps.

Additionally, the thickener included in the first content may include at least one selected from a group consisting of Gelatin, Gellan Gum, Guar Gum, Xanthan Gum, Locust Bean Gum, Alginic acid, Arabian Arabic Gum, Carrageenan, Pectin, Cellulose, Mannan, Carbomer, Sodium Polyacrylate, Polyacrylic Acid, Polyacrylate crosspolymer, Hydroxyethyl acrylate/Sodium acryloyldimethyl taurate Copolymer, Ammonium acryloyldimethyltaurate/vp copolymer, Sodium polyacryloyldimethyl taurate, Acrylate/C10-30 Alkyl acrylate Crosspolymer, Ammonium acryloyldimethyltaurate/beheneth-25methacrylate crosspolymer, Poly vinyl alcohol, glyceryl acrylate/acrylic acid copolymer, and Disteardimonium hectorite.

Hereinafter, the description will be directed to the activity of the enzyme when the components of the first content and the second content are mixed in the channel part 400 of the cosmetic container 10 to be described later.

**[Table 1] Second content**

| Component name | Weight |
|---|---|
| Purified water | To100 |
| Butylene Glycol | 20 |
| Propanediol | 20 |
| Xanthan gum | 0.2 |
| Hydroxyethyl acrylate/sodium acryloyldimethyl taurate copolymer | 0.5 |
| Ammonium acryloyldimethyltaurate/vp copolymer | 0.2 |
| Protease | 0.3 |

In Table 1, protease is an example of an enzyme; butylene glycol and propanediol are examples of polyol; and hydroxyethyl acrylate/sodium acryloyldimethyl taurate copolymer and ammonium acryloyldimethyltaurate/vp copolymer are examples of thickeners.

**[Table 2] First content**

| Component name | Weight |
|---|---|
| Purified water | To100 |
| Butylene Glycol | 3.00 |
| Dipropylene Glycol | 2.00 |
| Methylpropanediol | 1.00 |
| 1,2-Hexanediol | 2.00 |
| Dimethicone | 2.00 |
| PPG-13 -Decyltetradeceth-24 | 0.10 |
| polysorbate20 | 0.10 |
| Polyglyceryl-3 Methylglucose Distearate | 0.10 |
| Hydrogenated Lecithin | 0.05 |
| Xanthan gum | 0.05 |
| Acrylate/C10-30 Alkyl acrylate Crosspolymer | 0.10 |
| Sodium polyacryloyldimethyl taurate | 0.10 |
| Ammonium acryloyldimethyltaurate/vp copolymer | 0.15 |
| Glyceryl Caprylate | 0.10 |
| Ethylhexylglycerin | 0.05 |
| Disodium EDTA | 0.05 |

The components listed in Table 2 may be understood as general components for forming cosmetic material.

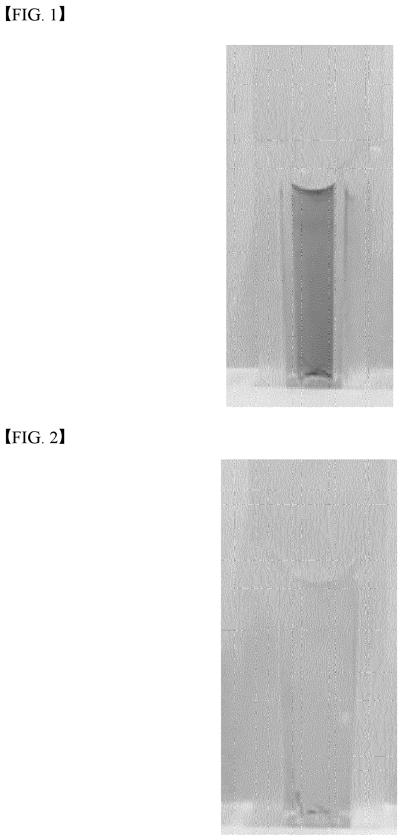

FIG. 1 is a photograph showing the degree of enzyme activation when the first content and the second content of Experimental Example 1 are mixed and discharged by means of a cosmetic container 10 to be described later, and FIG. 2 is a photograph of Comparative Example 1 showing the degree of enzyme activation when the first and second contents are mixed by means of a general mixer.

Referring to FIG. 1, it can be seen that when mixing the first content and the second content of Experimental Example 1 by means of a channel part 400 of the cosmetic container 10 to be described later, the activity of the enzyme is maintained.

The cosmetic composition of Experimental Example 1 shown in FIG. 1 may be shown in blue color, which may be understood as maintaining the activity of enzyme.

In contrast, referring to FIG. 2, it can be seen that when mixed with a general mixer the enzyme is destroyed.

The cosmetic composition of Comparative Example 1 shown in FIG. 2 may be shown in light yellow color, which may be understood as a state in which the enzyme is destroyed.

Here, a Homo Mixer was used as a general mixer, and for the enzyme activity measurement, the degree of destruction of the enzyme was measured by measuring the change in color caused by the substance produced when Keratin-Azure, as a substrate, is decomposed by the enzyme.

The blue color shown in FIG. 1 represents that the activity of the enzyme is maintained, and the liquid shown in FIG. 2 and having lost the blue color represents that the enzyme has been destroyed.

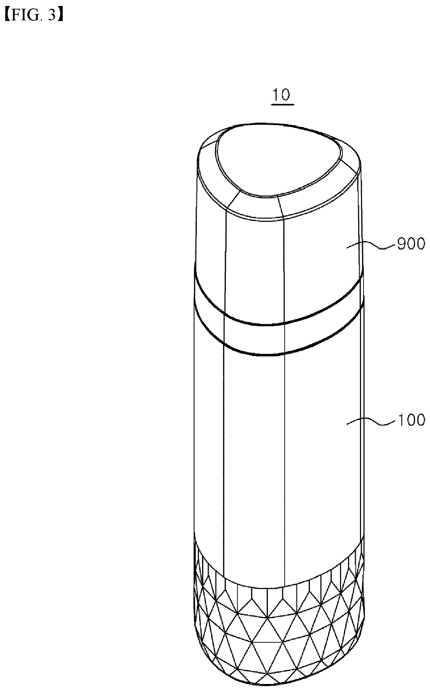

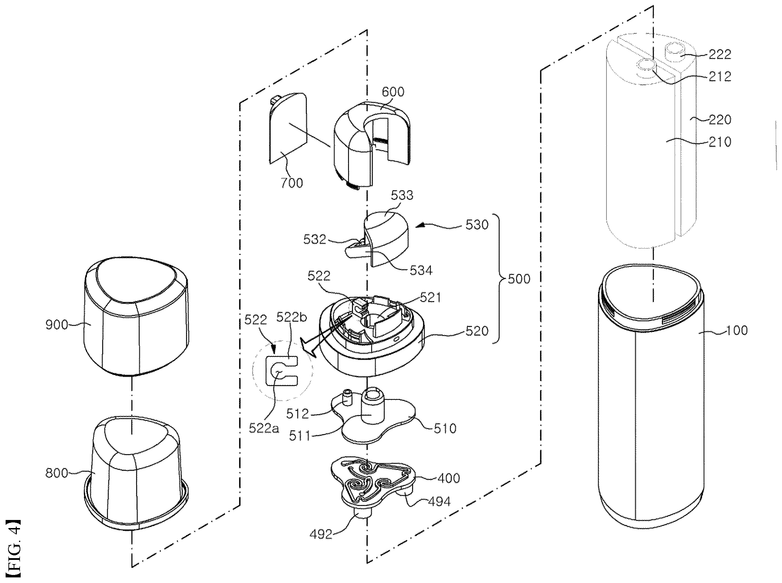

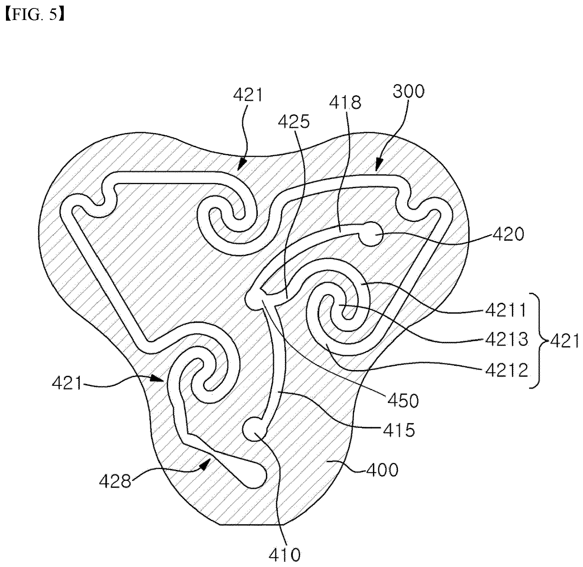

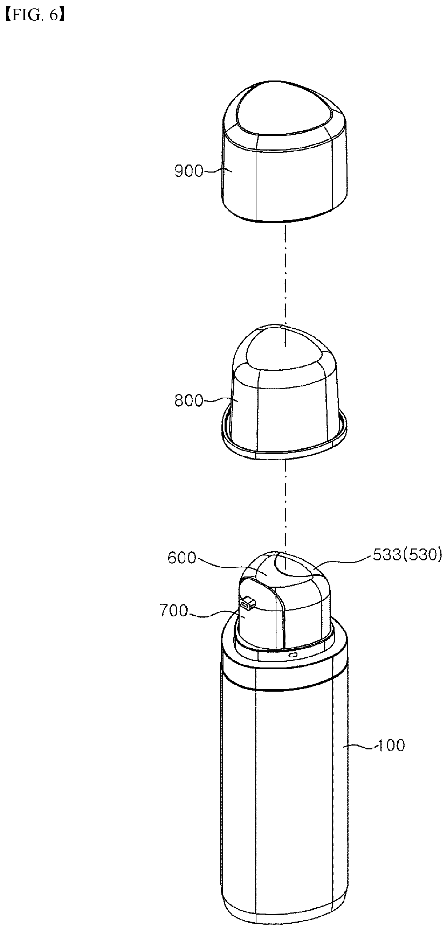

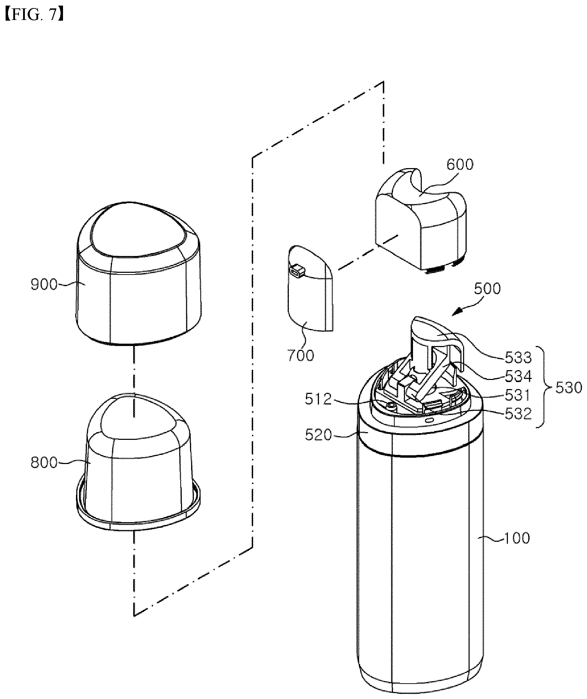

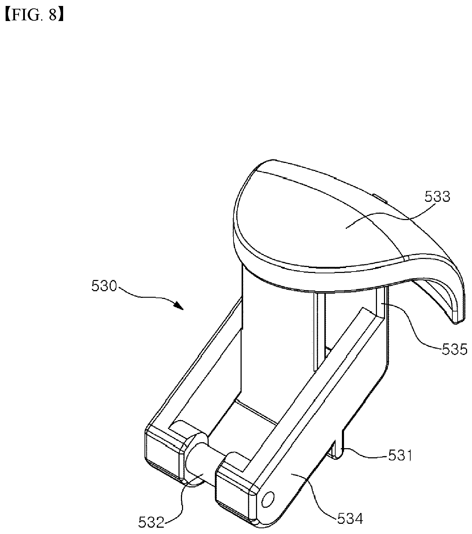

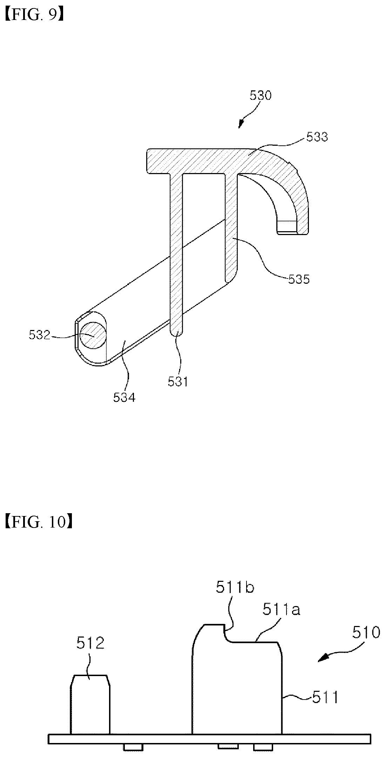

FIG. 3 is a perspective view schematically showing a cosmetic container 10 according to another embodiment of this disclosure, FIG. 4 shows an exploded perspective view of the cosmetic container 10 of FIG. 3, FIG. 5 is a cross-sectional view showing the microfluidic channel 300 formed in the channel part 400 of FIG. 4, FIG. 6 is a perspective view showing a state in which an inner cap 800 and an outer cap 900 are separated from the cosmetic container 10 of FIG. 3, FIG. 7 is an exploded perspective view showing the detailed configuration of an operation unit 500 in the cosmetic container 10 of FIG. 3, FIG. 8 is a perspective view showing a button lever 530 of the cosmetic container 10 of FIG. 7, FIG. 9 is a side cross-sectional view of the button lever 530 of FIG. 8, and FIG. 10 is a side view showing a pressurization panel of the operation unit 500 of FIG. 4.

Hereinafter, with reference to FIGS. 3 to 10, the cosmetic container 10 capable of storing the cosmetic composition for preventing the above-described enzyme damage will be described.

According to another embodiment of this disclosure, there may be provided a cosmetic container 10 including a first container 210 that provides a first space in which a first content can be stored; a second container 220 that provides a second space in which a second content can be stored; and a channel part 400 provided with a microfluidic channel 300 capable of forming a cosmetic material by mixing the first content and the second content.

Here, the cosmetic material produced by mixing the first content and the second content may be an emulsion or a solubilization cosmetic material as described above.

In addition, the Reynolds numbers of the first content, the second content and the cosmetic material (i.e., the mixture thereof), which pass through the microfluidic channel 300 of the channel part 400, may be provided equal to or less than 1.

When the Reynolds numbers become 1 or less during the process of mixing the first content and the second content in the microfluidic channel 300 of the channel part 400, it is possible to prevent the enzyme contained in the second content from being damaged by the vortex.

Here, the Reynolds number, which is the ratio of the force due to inertia and the force due to viscosity, can be understood as representing the relative dynamic relationship between these two types of forces under a given flow condition.

In other words, by optimally designing the viscosity of the thickener contained in the first content, the viscosity of the thickener contained in the second content, and the shape of the microfluidic channel 300 formed in the channel part 400, the enzyme contained in the second content can be prevented from being damaged during the process of passing through the microfluidic channel 300 of the channel part 400 while at the same time the first content and the second content can be mixed to form an emulsion or solubilization cosmetic material.

Additionally, the microfluidic channel 300 of the channel part 400 may be formed to have a thickness of 1 mm or less. In this case, the first content, the second content, and the cosmetic material (i.e., the mixture thereof) flowing through the microfluidic channel 300 form a laminar flow, thereby minimizing direct contact and preventing the enzyme from being destroyed.

When the height of the microfluidic channel 300 exceeds 1 mm, the Reynolds number (Re, inertial force/viscous force) may be increased, and the possibility of vortexing due to the inertial force may be increased, so that the destruction of the enzyme may occur.

Here, the height of the microfluidic channel 300 refers to a length measured in the vertical direction on the basis of FIG. 3.

Mixing two or more contents in the microfluidic channel 300 using only a physical force and a fluid motion principle without using external power may be defined as passive mixing.

In the case of mixing by the passive mixing, it is possible to induce the interfaces of the first content and the second content to overlap and mix, by which the emulsion can be provided in a state where turbulence does not occur in the fluids (the first content and the second content) passing through the microfluidic channel 300.

That is, in the microfluidic channel 300 of the channel part 400, the first content and the second content are mixed in a laminar flow form using passive mixing, to then produce an emulsion or a solubilization cosmetic, and thus it is possible to minimize or eliminate the possibility that enzymes in the second content pass through the interface therebetween and come into contact with the first content. Accordingly, the destruction of the enzyme in the emulsion or solubilization cosmetic material can be prevented.

Additionally, the fluid entry velocities of the first content, the second content, and the cosmetic material (i.e., mixture thereof) passing through the microfluidic channel 300 of the channel part 400 may be provided equal to or less than 0.9 m/s. The fluids (the first content, the second content, and the cosmetic material which is the mixture thereof) passing through the microfluidic channel 300 form a laminar flow by having such a speed range, thereby preventing the enzyme from being destroyed.

Here, the fluid entry velocity may be understood as the flow velocity at which the first content, the second content, and the cosmetic material (i.e., the mixture thereof) flow within the microfluidic channel 300.

The channel part 400 may include a plate provided in the form of a flat plate; and a microfluidic channel 300 which is formed inside the plate and is a flow path through which the first content and the second content pass.

Since the microfluidic channel 300 of the channel part 400 is positioned inside the flat plate-shaped plate, the entire microfluidic channel 300 may be positioned on the same plane inside the plate.

In this case, the cosmetic container 10 can be miniaturized by disposing the microfluidic channel 300 on the flat plate.

Additionally, the cross-section of the microfluidic channel 300 may be provided in a rectangular or circular shape.

The channel part 400 may include a first injection hole 410 for receiving the first content from the first container 210; a second injection hole 420 for receiving the second content from the second container 220; a confluence part 450 where the first content and the second content are joined to produce a cosmetic material; and a plurality of mixing parts 421 extending from the confluence part 450 to change the direction of progress of the first content, the second content, and the cosmetic material which is the mixture thereof (see FIG. 5).

Additionally, the microfluidic channel 300 constituting the channel part 400 may include a first connection flow path 415 connecting the first injection hole 410 and the confluence part 450; and a second connection flow path 418 connecting the second injection hole 420 and the confluence part 450.

Additionally, the channel part 400 may include a mixing part entry path 425 connecting the confluence part 450 and the first mixing part 421.

Additionally, after the third mixing part 421, a particle size adjusting part 428 may be provided. The particle size adjusting part 428 may allow the emulsion mixed in non-uniform sizes to be formed in a uniform size while passing through the plurality of mixing parts 421. In this embodiment, three mixing parts 421 are provided as an example, and the plurality of mixing parts 421 may be referred to as a first mixing part 421, a second mixing part 421, and a third mixing part 421, respectively in an order along direction in which the fluid flows.

Additionally, the bottom side of the channel part 400 may be provided with a first connection part 492 that can be connected to a first pumping part 212 of the first container 210; and a second connection part 494 that can be connected to a second pumping part 222 of the second container 220.

In this embodiment, there may be three mixing parts 421. When the number of mixing parts 421 exceeds three, a vortex may occur in the microfluidic channel 300 and destroy the enzyme.

Here, the microfluidic channel 300 may be understood as a continuous flow path including the confluence part 450, the mixing part 421, and the flow path connecting them.

The microfluidic channel easily contributes to enlarging the contact area between the first content and the second content, or to increasing the contact time. Additionally, the microfluidic channel can harden the interfacial film of the emulsified particles constituting the emulsion because the governing force of the surface tension is greater than in the macroscopic environment.

Additionally, the cosmetic container 10 of this embodiment may form an O/W emulsion or a W/O emulsion according to the ratio at which the first content stored in the first container 210 and the second content stored in the second container 220 are mixed. For example, if fluids included in the first content and the second content are an oil-based fluid and a water-based fluid, and they are mixed at such a mixing ratio that the amount of the water-based fluid is more than that of the oil-based fluid, the O/W emulsion can be produced, and in an opposite case, the W/O emulsion can be produced.

Additionally, the plate constituting the channel part 400 may be transparent, and in this case, the fluid flowing in the microfluidic channel may be visually visible to a user.

The mixing part 421 may change the moving direction of the fluid such as the rotational direction of the fluid by the flow path.

One mixing part 421 may be understood as having one or more flow paths each of which converts a turning direction.

The mixing part 421 may be disposed around the outer side of the confluence part 450. That is, when the plate forming the channel part 400 is viewed from the top (when viewed from the same viewpoint as in FIG. 5), the mixing part 421 may be disposed to surround the confluence part 450.

By disposing the mixing part 421 in the circumference region of the channel part 400 in this way, the length of the microfluidic channel 300 can be sufficiently long, so that a sufficient emulsification action can occur even in a small plate.

In the embodiment, the first mixing part 421 is described by way of example as being configured to rotate the entering fluid in one direction (in the embodiment, clockwise based on the drawing) and then rotate it in the other direction (in the embodiment, anticlockwise based on the drawing).

Specifically, the first mixing part 421 may include a first turning path 4211 which guides fluid so as to rotate in one direction, a second turning path 4212 which guides fluid so as to rotate in the other direction, and a direction conversion path 4213 which converts the rotating direction of the fluid between the first turning path 4211 and the second turning path 4212.

By this first mixing part 421, the first content and the second content are moved along the first turning path 4211 and rotated in one direction, and the rotating direction is converted in the direction conversion path 4213 to be rotated in the other direction, so that emulsions or solubilizing cosmetic material can be effectively produced.

Meanwhile, in the embodiment, the flow paths (microfluidic channels) formed in the channel part 400 may substantially form a single layer path.

The single layer path may be understood as a path in which height difference of flow paths is not involved in the mixing process of fluid.

Hereinafter, the cosmetic container 10 including the operation unit 500 which operates on the principle of leverage will be described in more detail.

The cosmetic container 10 for storing the cosmetic composition, which prevents enzyme damage, may be provided in such a configuration that it can mix the first content and the second content stored in the first container 210 and the second container 220 by introducing them into the microfluidic channel 300 of the channel part 400 and can easily discharge the cosmetic material mixed in the channel part 400.

For example, the cosmetic container 10 may further include a housing 100 surrounding the first container 210 and the second container 220 to form an outer appearance; and the operation unit 500 which moves the first content stored in the first container 210 and the second content stored in the second container 220 to the channel part 400 by a user's manipulation, and which provides an external force required to produce a cosmetic material by mixing the first content and the second content in the channel part 400, and to discharge the produced cosmetic material.

The operation unit 500 may be provided with an external force from the user, and the external force may be transmitted to the first pumping part 212 provided on the top of the first container 210 and to the second pumping part 222 provided on the top of the second container 220 via the channel part 400.

By pumping the first pumping part 212 and the second pumping part 222, the first content stored in the first container 210 and the second content stored in the second container 220 may be simultaneously introduced into the microfluidic channel 300 of the channel part 400 and mixed therein.

The operation unit 500 according to this embodiment may press the channel part 400 using the principle of leverage, and the channel part 400 may simultaneously press the first pumping part 212 of the first container 210 and the second pumping part 222 of the second container 220.

Thereby, it is possible to reduce the force required for a user to press the operation unit 500, and regardless of the position on the operation unit 500 which the user presses, the first container 210 and the second container 220 can be pressed with the same pressure. Accordingly, the discharge amounts of the first content and the second content can be made uniform.

In this embodiment, as the operation unit 500 is operated on the principle of leverage, one end of the operation unit 500 may become a rotatably fixed leverage point.

Additionally, the other end of the operation unit 500 located on a side opposite to the fixed leverage point becomes a force application point which the user presses, and when the user presses the force application point, the force application point can move while making a circular arc movement about the fixed leverage point.

Additionally, a predetermined point located between the fixed leverage point and the force application point may be a point of action, which may uniformly press the entire channel part 400.

When the entire channel part 400 is uniformly pressed, the same pressure may be applied to the first container 210 and the second container 220 disposed below the channel part 400.

Referring to FIGS. 4 and 7, the operation unit 500 may include a pressurization panel 510, a cover part 520, and a button lever 530.

The pressurization panel 510 may be positioned above the channel part 400, and may be configured to have the same planar shape as that of the channel part 400. The pressurization panel 510 may be coupled to the top surface of the channel part 400, and may seal the microfluidic channel 300 of the channel part 400 when coupled, so that leakage does not occur therebetween.

The pressurization panel 510 may have a central part 511 which is formed on its upper side, so that it can receives an external force from the point of action of the operation unit 500, and apply a uniform pressure to the channel part 400. The central part 511 may be provided as a structure that protrudes upwardly from the upper surface of the pressurization panel 510 so as to be in contact with the point of action. The detailed structure of the central part 511 will be described later in more detail.

Additionally, the pressurization panel 510 may include a discharge hole 512 which is connected (communicated) with the microfluidic channel 300 of the channel part 400, so that it discharges the emulsion or solubilization cosmetic material produced in the channel part 400 to the outside of the channel part 400.

The discharge hole 512 may be formed to extend upward by a predetermined height from one edge on the pressurization panel 510, and the discharge hole 512 may be communicated with the discharge part 700. The discharge part 700 may be provided to supply the user with an emulsion or solubilization cosmetic material which has been mixed.

The cover part 520 may be positioned above the pressurization panel 510 to cover and accommodate the pressurization panel 510 and the channel part 400. The cover part 520 may be detachably coupled to the top of the housing 100 so that it partially or entirely covers the pressurization panel 510 and the channel part 400. In this case, the pressurization panel 510, the channel part 400, the first container 210, and the second container 220 may be sequentially disposed under the cover part 520.

The cover part 520 may be provided with a through hole 521 which is formed in the center thereof so that the central part 511 of the pressurization panel 510 penetrates through the cover part 520 and protrudes upward. The through hole 521 may have a size and shape corresponding to the size and shape of the central part 511. Accordingly, when the pressurization panel 510 is disposed on the bottom side of the cover part 520, the central part 511 may pass through the through hole 521 of the cover part 520, and may protrude upward from the top of the cover part 520 by a predetermined height.

Additionally, the cover part 520 may include a hook part 522 to which one side of the button lever 530 is detachably coupled to form a fixed leverage point.

Referring to FIG. 4, the hook part 522 may include a pin hole 522a with one side opened, and a support part 522b providing an elastic force around the pin hole 522a.

Additionally, the button lever 530 may be rotatably connected to one side of the cover part 520 to form the fixed leverage point. This button lever 530 may serve as a lever.

The button lever 530 may be provided with a rib 531 which is formed at a point corresponding to the central part 511 of the pressurization panel 510 to provide the point of action between the force application point and the fixed leverage point.

As shown in FIGS. 8 and 9, the rib 531 protrudes downward by a predetermined length so that it can press the central part 511 of the pressurization panel 510. Therefore, when the user presses the button lever 530, the button lever 530 is rotated downward around a hinge part 532 as the fixed leverage point, and thus the rib 531 protruding downward from the position corresponding to the central part 511 presses the central part 511 so that the pressurization panel 510 presses the entire channel part 400 uniformly.

Specifically, the button lever 530 may further include the hinge part 532, a pressing part 533, and a connecting arm 534 in addition to the rib 531.

The hinge part 532 may be inserted into the hook part 522 of the cover part 520 so that the button lever 530 can be rotated around the hinge part 532 as the fixing leverage point. At this time, since the hinge part 532 is detachably inserted into the pin hole 522a of the hook part 522, it can be separated therefrom as needed.

Additionally, the pressing part 533 may be located on the upper side of the rib 531, and may serve as a force application point which receives an external force from the user. Additionally, when the user presses with his/her finger the pressing part 533 serving as the force application point, the pressing part 533 causes the button lever 530 to be rotated downward around the hinge part 532 serving as the fixed leverage point, and thus the rib 531 serving as the point of action of the button lever 530 moves downward and presses the central part 511, so that the external force is transmitted to the pressurization panel 510.

Additionally, the connecting arm 534 connects the rib 531 and the hinge part 532 to each other so that the force of the pressing part 533 can be transmitted to the hinge part 532, and the rib 531 can be stably supported.

Here, as shown in FIG. 9, the button lever 530 may further include a sub-rib 535.

The sub-rib 535 is parallel to and spaced away from the rib 531, and connects the pressing part 533 and the connecting arm 534 but does not protrude downward from the connecting arm 534 so that it does not interfere when the rib 531 presses the central part 511. The sub-rib 535 serves to reinforce the rib 531 connected between the pressing part 533 and the connecting arm 534, so that it is possible to prevent the pressing part 533 and the rib 531 from being damaged when the user presses the pressing part 533.

The central part 511 of the pressurization panel 510 will be described in more detail with reference to FIG. 10.

The central part 511 may be provided as a structure capable of receiving an external force from the rib 531 of the button lever 530 while being in contact with the rib 531 serving as the point of action of the lever, and of transmitting the external force to the pressurization panel 510.

The central part 511 protrudes upward by a predetermined height from the upper surface of the pressurization panel 510, and may be provided in the form of a hollow cylinder having an empty central axle part therein. Additionally, on the upper surface of the central part 511, a flat slip part 511a may be provided so that the rib 531 can slide thereon while being in contact with it. Also, at the end of the slip part 511a, a blocking part 511b that protrudes more upward than the slip part 511a may be formed so that the rib 531 can be caught by it.

That is, the central part 511 may be formed to include the slip part 511a and the blocking part 511b. Accordingly, when the user presses the pressing part 533, the rib 531 applies an external force directed downward to the slip part 511a. At the same time, as the pressing part 533 serving as the force application point performs a circular arc motion, the rib 531 in contact with the slip part 511a applies not only a direct downward force to the slip part 511a but also performs a sliding motion on the slip part 511a toward the blocking part 511b. Additionally, the rib 531 is caught by the blocking part 511b, limiting the movement of the button lever 530, and accordingly, the one-time pressing operation of the pressing part 533 is finished, and it is possible to prevent the button lever 530 from being damaged by an external force.

As shown in FIG. 7, a button decoration part 600 may be provided as a structure that can expose the pressing part 533 of the button lever 530 to the outside so that the user can operate the pressing part 533, and can allow the discharge part 700 to be disposed on one side thereof. After the button decoration part 600 is installed on the cover part 520, an inner cap 800 and an outer cap 900 may be sequentially fitted thereon to cover and protect the internal components.

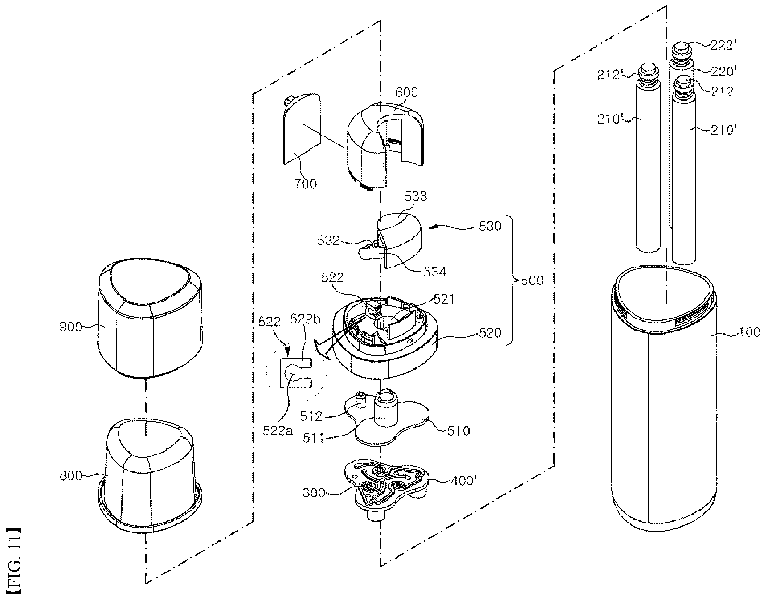

FIG. 11 represents an exploded perspective view of a cosmetic container according to another embodiment of this disclosure.

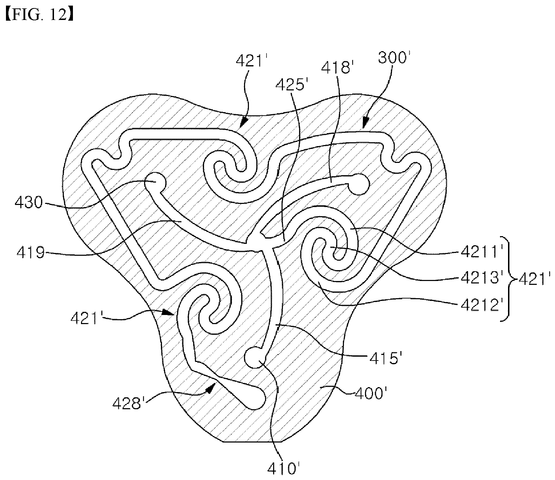

FIG. 12 is a diagram representing a cross-section of a channel part 400' provided in the cosmetic container of FIG. 11.

Hereinafter, a cosmetic container according to another embodiment of this disclosure will be described with reference to FIGS. 11 and 12.

The embodiment shown in FIGS. 3 to 10 has been described as an example in which two containers 210 and 220 are provided. Compared to the cosmetic container shown in FIGS. 3 to 10, the cosmetic container shown in FIGS. 11 and 12 is different in that it includes three containers, and an additional injection hole 430 which is further provided in the channel part 400'. So, differences will be mainly described, and descriptions of the above-described embodiments and reference numerals will be used for the same components.

Referring to FIGS. 11 and 12, the cosmetic container according to another embodiment of this disclosure may include a plurality of first containers 210' each of which provides a first space in which a first content can be stored; a second container 220' that provides a second space in which a second content can be stored; and a channel part 400' provided with a microfluidic channel 300' capable of forming a cosmetic material by mixing the first content and the second content.

As described above, the Reynolds numbers of the first content, the second content and the cosmetic material (i.e., the mixture thereof), which pass through the microfluidic channel 300' of the channel part 400', may be provided equal to or less than 1.

In this embodiment, two first containers 210' and one second container 220 are, by way of example, provided. That is, the first content may be divided and stored in the two first containers 210', and the second content may be stored in one second container 220'.

In this case, the mixing ratio of the first content and the second content may be adjusted according to the number and storage capacity of the first container 210' and the second container 220'.

The channel part 400' according to another embodiment of this disclosure may include a first injection hole 410' for receiving the first content from the first container 210'; an additional injection hole 430 for receiving the first contents from another first container 210'; a second injection hole 420' for receiving the second content from the second container 220'; a confluence part 450' where the first content and the second content are joined to produce a cosmetic material; and a plurality of mixing parts 421' extending from the confluence part 450' to change the direction of progress of the first content, the second content, and the cosmetic material which is the mixture thereof (see FIG. 12).

Additionally, the microfluidic channel 300' constituting the channel part 400' may include a first connection flow path 415' connecting the first injection hole 410' and the confluence part 450'; a second connection flow path 418' connecting the second injection hole 420' and the confluence part 450'; and a third connection flow path 419 connecting the additional injection hole 430 and the confluence part 450'.

Additionally, the channel part 400' may include a mixing part entry path 425 connecting the confluence part 450' and the first mixing part 421'.

Additionally, after the third mixing part 421', a particle size adjusting part 428' may be provided.

While until now the cosmetic composition for preventing enzyme damage and the cosmetic container 10 storing the same according to embodiments of this disclosure have been described as concrete embodiments, these are just exemplary embodiments, and this disclosure should be construed in a broadest scope based on the fundamental technical ideas disclosed herein, rather than as being limited to them. By combining or replacing a part or parts of embodiments disclosed herein, the ordinary skilled in the art may carry out an embodiment which is not explicitly described herein, and however, it should be noted that it shall not depart from the scope of this disclosure. Besides, the ordinary skilled in the art may easily change or modify embodiments disclosed herein based on this disclosure, and however, it is obvious that such changes or modifications also fall within the scope of this disclosure.

**[Reference Sign List]**

| | | | |
|---|---|---|---|
| 10: | cosmetic container | 100: | housing |
| 210, 210': | first container | 220, 220': | second container |
| 300, 300': | microfluidic channel | 400, 400': | channel part |
| 421, 421': | mixing part | 500: | operation unit |

## Claims

1. A cosmetic composition capable of preventing enzyme damage, the composition comprising:
a first content disposed in a first space;
a second content which is disposed in a second space separated from the first space, and which includes an enzyme and a polyol,
wherein a cosmetic material is produced by mixing the first content and the second content.

2. The composition of claim 1, wherein the polyol includes at least one selected from a group consisting of ethylene glycol, propylene glycol, dipropylene glycol, 1,3-butylene glycol, diglycerin, 1,3-propanediol, glycerin, methylpropanediol, ethylhexanediol, 1,2-hexanediol, isoprene glycol, ethylalcohol, pentylene glycol, and isopentyldiol.

3. The composition of claim 1, wherein the polyol is provided in an amount of 40% or more based on the total weight of the second content.

4. The composition of claim 1, wherein the enzyme is provided as a protease.

5. The composition of claim 4, wherein the protease includes at least one selected from a group consisting of cysteine protease, serine protease, threonine protease, glutamate protease, aspartic acid protease, metalloprotease, asparagine protease, keratinase, papain, and lipase.

6. The composition of claim 1, wherein the second content further includes a thickener having a viscosity of 600 cps to 8,000 cps.

7. The composition of claim 6, wherein the thickener includes at least one selected from a group consisting of gelatin, gellan gum, guar gum, xanthan gum, locust bean gum, alginic acid, arabian gum, carrageenan, pectin, cellulose, mannan, carbomer, sodium polyacrylate, polyacrylic acid, polyacrylate crosspolymer, hydroxyethyl acrylate/sodium acryloyldimethyl taurate copolymer, ammonium acryloyldimethyltaurate/vp copolymer, sodium polyacryloyldimethyl taurate, acrylate/C10-30 alkyl acrylate crosspolymer, ammonium acryloyldimethyltaurate/beheneth-25 methacrylate crosspolymer, poly vinyl alcohol, and glyceryl acrylate/acrylic acid copolymer.

8. The composition of claim 1, wherein the first content includes at least one of a moisturizing agent, a surfactant, and a thickener.

9. The composition of claim 1, wherein the first content includes a surfactant, and the second content including the enzyme does not include a surfactant.

10. The composition of claim 1, wherein the cosmetic material produced by mixing the first content and the second content includes emulsion or solubilization cosmetic material.

11. A cosmetic container (10) for storing a cosmetic composition according to any one of claims 1 to 10, the container comprising:
a first container (210) providing a first space in which a first content can be stored;
a second container (220) providing a second space in which a second content can be stored; and
a channel part (400) provided with a microfluidic channel (300) capable of forming a cosmetic material by mixing the first content and the second content,
wherein the Reynolds numbers of the first content, the second content, and the cosmetic material which pass through the microfluidic channel (300) of the channel part (400) are provided to be 1 or less, the cosmetic material being a mixture of the first and second contents.

12. The cosmetic container of claim 11, wherein the microfluidic channel (300) has a height of 1 mm or less.

13. The cosmetic container of claim 11, wherein the fluid entry velocities of the first content, the second content, and the cosmetic material which pass through the microfluidic channel (300) of the channel part (400) are provided to be 0.9 m/s or less.

14. The cosmetic container of claim 11, wherein the channel part includes:
a first injection hole (410) for receiving the first content from the first container;
a second injection hole (420) for receiving the second content from the second container;
a confluence part (450) where the first content and the second content are joined to produce the cosmetic material; and
a plurality of mixing parts (421) extending from the confluence part (450) so as to change the progress directions of the first content, the second content, and the cosmetic material.

15. The cosmetic container of claim 14, wherein a number of the mixing parts (421) is three.
